# EUROPEAN PATENT APPLICATION

(11) **EP 1 331 683 A2**
(43) Date of publication of application: **30.07.2003**
(21) Application number: 03001616.6
(22) Date of filing: 24.01.2003
(51) Int. Cl.: H01M 4/48

(54) **Cathode active material coated with a metal oxide for incorporation into a lithium electrochemical cell**

(30) Priority: 24.01.2002 US 351947 P
(71) Applicant: Wilson Greatbatch Technologies, Inc., Clarence, New York 14031 (US)
(72) Inventor: Leising, Randolph, Williamsville, New York 14221 (US); Takeuchi, Esther S., East Amherst, New York 14051 (US)
(74) Representative: Koepe, Gerd L., Dipl.-Chem.

(57) **Abstract**

An improved cathode material for nonaqueous electrolyte lithium electrochemical cell is described. The preferred active material is silver vanadium oxide (SVO) coated with a protective layer of an inert metal oxide (MₓO_{y}) or lithiated metal oxide (LiₓM_{y}O_{z}). The SVO core provides high capacity and rate capability while the protective coating reduces reactivity of the active particles with electrolyte to improve the long-term stability of the cathode.

## Description

### CROSS-REFERENCE TO RELATED APPLICATION

This application claims priority based on provisional application Serial No. 60/351,947, filed January 24, 2002.

### BACKGROUND OF THE INVENTION

### 1. Field Of Invention

This invention relates to the conversion of chemical energy to electrical energy. In particular, the present invention relates to preparation of an improved cathode material for lithium electrochemical cells containing silver vanadium oxide (SVO) or copper silver vanadium oxide (CSVO) coated with a protective layer of an inert metal oxide (MₓO_{y}) or lithiated metal oxide (LiₓM_{y}O_{z}). For example, the new active material contains a core of ε-phase SVO providing the cell with relatively high capacity and rate capability. A protective coating of MₓO_{y} or LiₓM_{y}O_{z} on the active material reduces particle reactivity with electrolyte and improves the long-term stability of the cathode. Improved long-term stability of the cathode active material translates into increased life upon incorporation into a lithium electrochemical cell. An exemplary application is having the cell power an implantable cardiac defibrillator, where the cell may run under a light load for extended periods of time interrupted by high rate pulse discharge.

### 2. Prior Art

As is well known by those skilled in the art, an implantable cardiac defibrillator is a device that requires a power source for a generally medium rate, constant resistance load component provided by circuits performing such functions as, for example, the heart sensing and pacing functions. From time-to-time, the cardiac defibrillator may require a generally high rate, pulse discharge load component that occurs, for example, during charging of a capacitor in the defibrillator for the purpose of delivering an electrical shock to the heart to treat tachyarrhythmias, the irregular, rapid heartbeats that can be fatal if left uncorrected.

It is generally recognized that for lithium cells, silver vanadium oxide (SVO) and, in particular, ε-phase silver vanadium oxide (AgV₂O_{5.5}), is preferred as the cathode active material. U.S. Patent Nos. 4,310,609 and 4,391,729, both to Liang et al., disclose the preparation of ε-phase SVO as a cathode material for use in a nonaqueous electrolyte electrochemical cell. These patents describe the preparation of silver vanadium oxide through the use of a thermal decomposition reaction of silver nitrate with vanadium oxide (V₂O₅) at a maximum temperature of -360°C. The Liang et al. patents are assigned to the assignee of the present invention and incorporated herein by reference.

Silver vanadium oxide is preferred for cardiac defibrillators because of its relatively high rate capability. For example, U.S. Patent No. 4,830,940 to Keister et al. discloses a primary cell containing silver vanadium oxide for delivering high current pulses with rapid recovery, high capacity and low self-discharge. The Keister et al. patent is assigned to the assignee of the present invention and incorporated herein by reference.

A discussion related to the surface modification of inorganic particles is found in U.S. Patent No. 3,905,936 to Hawthorne. This patent describes the surface treatment of active particles with chemically bonded organic aluminum derivatives of the formula (RO)ₙAlR'₃₋ₙ. The chemically bonded layer confers improved mechanical properties on the active material. However, these coatings were applied at relatively low temperatures and were not heat treated to decompose the Al coating to a metal oxide.

U.S. Patent 6,296,972 B1 to Hong et al. discloses coating a NiO cathode used for a molten carbonate fuel cell with LiCoO₂ prepared by a sol-gel process. A sol is prepared using stoichiometric amounts of lithium and cobalt salts in a solvent with or without adding a chelating agent. The NiO electrode is impregnated with the sol and the electrode dried under vacuum and calcined. The heat treatment (calcining) temperature is not specified in this patent, however, LiCoO₂ materials are typically heat treated to about 700°C to about 1000°C to form this material.

In the paper: "Modification of LiₓNi_{1-y}Co_{y}O₂ By Applying a Surface Coating of MgO", Kweon, H.J.; Kim, S.J.; Park, D.G. *J. Power Sources* 2000, *88*, 255-261, the authors described coating a LiₓNi_{1-y}Co_{y}O₂ cathode material with a surface layer of MgO. The modified cathode active material displayed improved cycle reversibility for rechargeable lithium-ion cells. The LiₓNi_{1-y}Co_{y}O₂ particles were coated with a magnesia xerogel [Mg(OMe)₂] and heated at 750°C for 12 hours to form the protective MgO coating.

### SUMMARY OF THE INVENTION

Accordingly, the present invention provides a process for preparing a composite SVO cathode material containing a SVO (ε-phase Ag₂V₄O₁₁ or γ-phase Ag_{0.8}V₂O_{5.4}) or CSVO core coated with a protective metal oxide or lithiated metal oxide surface layer. The coating can include SnO₂, SiO₂, Al₂O₃, ZrO₂, B₂O₃, MgO, LiCoO₂, MnO₂, LiMnOₓ, and mixtures thereof. These materials are preferably applied via a sol-gel process to provide a thin coating over the SVO or CSVO core. This results in a new composite material with improved performance over prior art cathode active materials. In particular, voltage delay and Rdc build-up during long-term cell discharge are reduced since the cathode active material is isolated from the electrolyte.

These and other objects of the present invention will become increasingly more apparent to those skilled in the art by reference to the following description and to the appended drawings.

### BRIEF DESCRIPTION OF THE DRAWINGS

Fig. 1 is a flow chart illustrating the processing steps for coating a particle of active material with a metal oxide according to the present invention.
Fig. 2 is a schematic of a patient P provided with an implantable medical device 100.
Fig. 3 is an enlarged schematic of the indicated area in Fig. 2 particularly showing the control circuitry 104, the electrochemical cell 106 and capacitor 108 for the medical device 100 connected to the patient's heart H.

### DETAILED DESCRIPTION OF THE PREFERRED EMBODIMENTS

As used herein, the term "pulse" means a short burst of electrical current of significantly greater amplitude than that of a pre-pulse current immediately prior to the pulse. A pulse train consists of at least two pulses of electrical current delivered in relatively short succession with or without open circuit rest between the pulses. An exemplary pulse train may consist of four 10-second pulses (23.2 mA/cm²) with a 15 second rest between each pulse. A typically used range of current densities for cells powering implantable medical devices is from about 15 mA/cm² to about 50 mA/cm², and more preferably from about 18 mA/cm² to about 35 mA/cm², Typically, a 10 second pulse is suitable for medical implantable applications. However, it could be significantly shorter or longer depending on the specific cell design and chemistry.

An electrochemical cell that possesses sufficient energy density and discharge capacity required to meet the vigorous requirements of implantable medical devices comprises an anode of a metal selected from Groups IA, IIA and IIIB of the Periodic Table of the Elements. Such anode active materials include lithium, sodium, potassium, etc., and their alloys and intermetallic compounds including, for example, Li-Si, Li-Al, Li-B and Li-Si-B alloys and intermetallic compounds. The preferred anode comprises lithium. An alternate anode comprises a lithium alloy such as a lithium-aluminum alloy. The greater the amounts of aluminum present by weight in the alloy, however, the lower the energy density of the cell.

The form of the anode may vary, but preferably the anode is a thin metal sheet or foil of the anode metal, pressed or rolled on a metallic anode current collector, i.e., preferably comprising titanium, titanium alloy or nickel, to form an anode component. Copper, tungsten and tantalum are also suitable materials for the anode current collector. In the exemplary cell of the present invention, the anode component has an extended tab or lead of the same material as the anode current collector, i.e., preferably nickel or titanium, integrally formed therewith such as by welding and contacted by a weld to a cell case of conductive metal in a case-negative electrical configuration. Alternatively, the anode may be formed in some other geometry, such as a bobbin shape, cylinder or pellet to allow an alternate low surface cell design.

The electrochemical cell of the present invention further comprises a cathode of electrically conductive material that serves as the other electrode of the cell. The cathode is preferably of solid materials comprising a metal element, a metal oxide, a mixed metal oxide and a metal sulfide, and combinations thereof. The cathode active material is formed by the chemical addition, reaction, or otherwise intimate contact of various metal oxides, metal sulfides and/or metal elements, preferably during thermal treatment, sol-gel formation, chemical vapor deposition or hydrothermal synthesis in mixed states. The active materials thereby produced contain metals, oxides and sulfides of Groups, IB, IIB, IIIB, IVB, VB, VIB, VIIB and VIII, which includes the noble metals and/or other oxide and sulfide compounds. A preferred cathode active material is a reaction product of at least silver and vanadium.

One preferred mixed metal oxide has the general formula SMₓV₂O_{y} where SM is a metal selected from Groups IB to VIIB and VIII of the Periodic Table of Elements, wherein x is about 0.30 to 2.0 and y is about 4.5 to 6.0 in the general formula. By way of illustration, and in no way intended to be limiting, one exemplary cathode active material comprises silver vanadium oxide having the general formula AgₓV₂O_{y} in any one of its many phases, i.e., β-phase silver vanadium oxide having in the general formula x = 0.35 and y = 5.8, γ-phase silver vanadium oxide having in the general formula x = 0.74 and y = 5.37 and ε-phase silver vanadium oxide having in the general formula x = 1.0 and y = 5.5, and combinations and mixtures of phases thereof. For a more detailed description of such cathode active materials reference is made to the previously discussed Liang et al. patents.

Another preferred composite metal oxide cathode material includes V₂O_{z} wherein z ≤ 5 combined with Ag₂O with silver in either the silver(II), silver(I) or silver(0) oxidation state and CuO with copper in either the copper(II), copper(I) or copper(0) oxidation state to provide the mixed metal oxide having the general formula CuₓAg_{y}V₂O_{z}, (CSVO). Thus, the composite cathode active material may be described as a metal oxide-metal oxide-metal oxide, a metal-metal oxide-metal oxide, or a metal-metal-metal oxide and the range of material compositions found for CuₓAg_{y}V₂O_{z} is preferably about 0.01 ≤ z ≤ 6.5. Typical forms of CSVO are Cu_{0.16}Ag_{0.67}V₂O_{z} with z being about 5.5 and Cu_{0.5}Ag_{0.5}V₂O_{z} with z being about 5.75. The oxygen content is designated by z since the exact stoichiometric proportion of oxygen in CSVO can vary depending on whether the cathode material is prepared in an oxidizing atmosphere such as air or oxygen, or in an inert atmosphere such as argon, nitrogen and helium. For a more detailed description of this cathode active material reference is made to U.S. Patent Nos. 5,472,810 to Takeuchi et al. and 5,516,340 to Takeuchi et al., both of which are assigned to the assignee of the present invention and incorporated herein by reference. In addition to silver vanadium oxide and copper silver vanadium oxide, V₂O₅, MnO₂, LiCoO₂, LiNiO₂, LiMnO₂, LiMn₂O₄, TiS₂, Cu₂S, FeS, FeS₂, Ag₂O, Ag₂O₂, CuF, Ag₂CrO₄, copper oxide, copper vanadium oxide, and mixtures thereof are useful as the cathode active material.

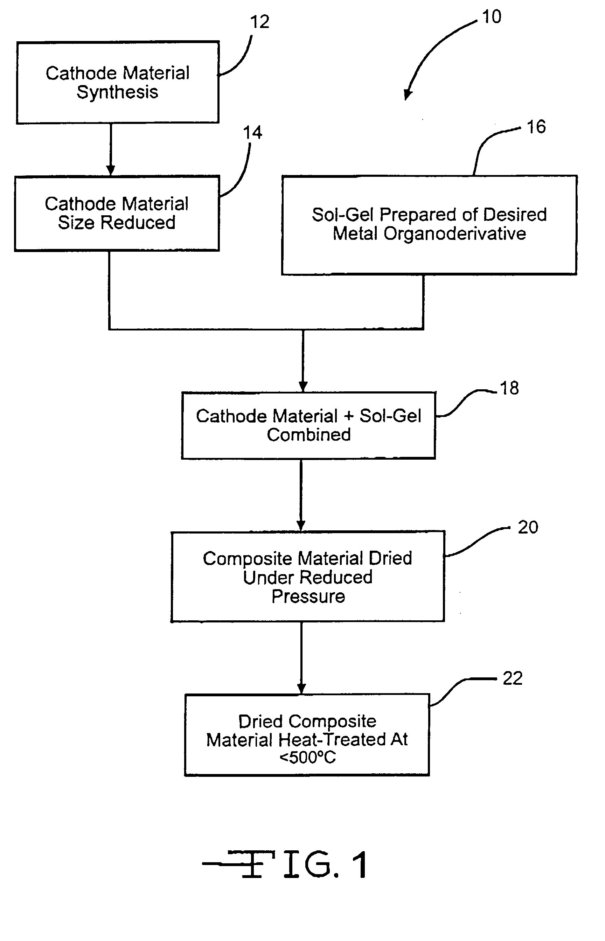

Fig. 1 shows a flow chart that illustrates the process 10 used to form the metal oxide or lithiated metal oxide coated SVO or CSVO particles according to the present invention. The process begins with 12 of the cathode active material. In the case of SVO, the active material can be prepared according to any known synthesis method. These include the synthesis techniques described in U.S. Patent Nos. 4,016,338 to Lauck, 4,158,722 to Lauck et al., 4,310,609 to Liang et al., 4,391,729 to Liang et al., 4,542,083 to Cava et al., 4,675,260 to Sakurai et al., 4,751,157 to Uchiyama et al., 4,751,158 to Uchiyama et al., 4,803,137 to Miyazaki et al., 4,830,940 to Keister et al., 4,964,877 to Keister et al., 4,965,151 to Takeda et al., 5,194,342 to Bito et al., 5,221,453 to Crespi, 5,298,349 to Takeuchi, 5,389,472 to Takeuchi et al., 5,545,497 to Takeuchi et al., 5,458,997 to Crespi et al., 5,472,810 to Takeuchi et al., 5,498,494 to Takeuchi et al., 5,498,495 to Takeda et al., 5,512,214 to Koksbang, 5,516,340 to Takeuchi et al., 5,558,680 to Takeuchi et al., 5,567,538 to Oltman et al., 5,670,276 to Takeuchi et al., 5,695,892 to Leising et al., 5,895,733 to Crespi et al., 5,955,218 to Crespi et al., 6,093,506 to Crespi et al., 6,130,055 to Crespi et al., and 6,413,669 to Takeuchi et al. Prior art synthesis for SVO are also described in Leising, R. A.; Takeuchi, E. S. *Chem. Mater.* 1993, 5, 738-742 and Leising, R. A.; Takeuchi, E. S. *Chem. Mater*. 1994, 6, 489-495. The latter Leising et al. publication describes a preferred method for the synthesis of SVO with the caveat that the temperature is less than 500°C to fully form the material. These patents and publications are incorporated herein by reference.

Next, the particle size of the cathode active material is reduced in step 14. This increases the material's surface area, which is beneficial for improved discharge efficiency. Several means are contemplated for reducing the size of the active particles including using a mortar and pestle, a ball mill, jet-mill, or by attrition. In addition, the SVO or CSVO materials may be used directly without particle size reduction.

A sol-gel solution 16 containing an organic derivative of the desired coating metal is prepared. The sol-gel solution can either be an aqueous or a non-aqueous based solution. Aqueous solutions include water and a minor amount of lithium hydroxide to bring the solution to a basic pH. Nonaqueous solutions are essentially alcohol based with methanol, ethanol, isopropyl and isobutyl being preferred. Useful metals for this purpose include aluminum, boron, cobalt, magnesium, manganese, silicon, tin, and zirconium. Lithium salts of these metals may also be added to the sol-gel solution 16 to produce a lithiated metal oxide coating. Either the SVO or CSVO material, or both, is then added to the sol-gel solution to form a mixture in step 18. In this step, it is important to carefully control the ratio of SVO or CSVO to sol-gel. Preferably, the solution contains, by weight, a ratio of coating material to active material in a range of about 1:3 to about 1:20, 1:5 being preferred. The resulting coated cathode active material is dried in step 20 under a reduced pressure in a range of about 20 inches of Hg. to about 50 inches of Hg., preferably about 30 inches of Hg., to remove the carrier solvent from the sol-gel.

The dried coated material is heat-treated in step 22 to form a metal oxide or lithiated metal oxide coating on the SVO or CSVO particles. The heat treatment step is critical to controlling the composition of the product. The heating range is about 200°C to about 500°C for a time of about 10 minutes to about 6 hours. Longer heating are required for lower temperatures. The maximum heating temperature is preferably below about 500°C. The protective coatings have the general formula of MₓO_{y} or LiₓM_{y}O_{z} wherein M is selected from the group consisting of Al, B, Mg, Mn, Si, Sn, and Zr. In the formula MₓO_{y}, x = 1 or 2 and y = 1 to 3 while in the formula LiₓM_{y}O_{z}, x = 1, y = 1 or 2 and z = 1 to 4. Exemplary coatings for SVO or CSVO include SnO₂, SiO₂, Al₂O₃, ZrO₂, B₂O₃, MgO, MnO₂, LiCoO₂, LiMnₓO_{y}, and mixtures thereof.

Unlike the prior art coated cathode preparations of the previously described Hawthorne and Hong et al. patents, relatively high temperatures (>500°C) produce poor SVO or CSVO cathode active materials regardless of whether the material is being coated, or not. The amount of time the composite material is heated is also important in determining the final product. Relatively long reaction times are to be avoided because they promote ion diffusion of metal atoms from the coating to migrate to the SVO or CSVO core, as ion diffusion is particularly rapid in these materials. Thus, the time and temperature parameters are key specific factors related to this invention.

Before fabrication into an electrode structure for incorporation into an electrochemical cell according to the present invention, the cathode active material prepared as described above is preferably mixed with a binder material such as a powdered fluoro-polymer, more preferably powdered polytetrafluoroethylene or powdered polyvinylidene flouride present at about 1 to about 5 weight percent of the cathode mixture. Further, up to about 10 weight percent of a conductive diluent is preferably added to the cathode mixture to improve conductivity. Suitable materials for this purpose include acetylene black, carbon black and/or graphite or a metallic powder such as powdered nickel, aluminum, titanium and stainless steel. The preferred cathode active mixture thus includes a powdered fluoro-polymer binder present at about 3 weight percent, a conductive diluent present at about 3 weight percent and about 94 weight percent of the cathode active material.

Cathode components for incorporation into an electrochemical cell according to the present invention are prepared by rolling, spreading or pressing the cathode active material onto a suitable current collector selected from the group consisting of stainless steel, titanium, tantalum, platinum, aluminum, gold, nickel, and alloys thereof. The preferred current collector material is titanium, and most preferably the titanium cathode current collector has a thin layer of graphite/carbon paint applied thereto. Cathodes prepared as described above may be in the form of one or more plates operatively associated with at least one or more plates of anode material, or in the form of a strip wound with a corresponding strip of anode material in a structure similar to a "jellyroll".

The cathode current collector is connected to a terminal insulated from the cell casing (not shown) by a suitable glass-to-metal seal. This describes a case-negative cell design, which is the preferred form of the present invention cell. The cell can also be built in a case-positive design with the cathode current collector contacted to the casing and the anode current collector connected to a terminal lead insulated from the casing. In a further embodiment, the cell is built in a case-neutral configuration with both the anode and the cathode connected to respective terminal leads insulated from the casing. These terminal constructions are well known by those skilled in the art.

In order to prevent internal short circuit conditions, the cathode is separated from the Group IA, IIA or IIIB anode by a suitable separator material. The separator is of electrically insulative material, and the separator material also is chemically unreactive with the anode and cathode active materials and both chemically unreactive with and insoluble in the electrolyte. In addition, the separator material has a degree of porosity sufficient to allow flow there through of the electrolyte during the electrochemical reaction of the cell. Illustrative separator materials include fabrics woven from fluoropolymeric fibers including polyvinylidine fluoride, polyethylenetetrafluoroethylene, and polyethylenechlorotrifluoroethylene used either alone or laminated with a fluoropolymeric microporous film, non-woven glass, polypropylene, polyethylene, glass fiber materials, ceramics, polytetrafluoroethylene membrane commercially available under the designation ZITEX (Chemplast Inc.), polypropylene/polyethylene membrane commercially available under the designation CELGARD (Celanese Plastic Company, Inc.), a membrane commercially available under the designation DEXIGLAS (C.H. Dexter, Div., Dexter Corp.), and a polyethylene membrane commercially available from Tonen Chemical Corp.

The electrochemical cell of the present invention further includes a nonaqueous, ionically conductive electrolyte that serves as a medium for migration of ions between the anode and the cathode electrodes during the electrochemical reactions of the cell. The electrochemical reaction at the electrodes involves conversion of ions in atomic or molecular forms that migrate from the anode to the cathode. Thus, nonaqueous electrolytes suitable for the present invention are substantially inert to the anode and cathode materials, and they exhibit those physical properties necessary for ionic transport, namely, low viscosity, low surface tension and wettability.

A suitable electrolyte has an inorganic, ionically conductive salt dissolved in a mixture of aprotic organic solvents comprising a low viscosity solvent and a high permittivity solvent. In the case of an anode comprising lithium, preferred lithium salts that are useful as a vehicle for transport of lithium ions from the anode to the cathode include LiPF₆, LiBF₄, LiAsF₆, LiSbF₆, LiClO₄, LiO₂, LiAlCl₄, LiGaCl₄, LiC(SO₂CF₃)₃, LiN(SO₂CF₃)₂, LiSCN, LiO₃SCF₃, LiC₆F₅SO₃, LiO₂CCF₃, LiSO₆F, LiB(C₆H₅)₄, LiCF₃SO₃, and mixtures thereof.

Low viscosity solvents useful with the present invention include esters, linear and cyclic ethers and dialkyl carbonates such as tetrahydrofuran (THF), methyl acetate (MA), diglyme, trigylme, tetragylme, dimethyl carbonate (DMC), 1,2-dimethoxyethane (DME), 1,2-diethoxyethane (DEE), 1-ethoxy,2-methoxyethane (EME), ethyl methyl carbonate, methyl propyl carbonate, ethyl propyl carbonate, diethyl carbonate, dipropyl carbonate, and mixtures thereof, and high permittivity solvents include cyclic carbonates, cyclic esters and cyclic amides such as propylene carbonate (PC), ethylene carbonate (EC), butylene carbonate, acetonitrile, dimethyl sulfoxide, dimethyl formamide, dimethyl acetamide, γ-valerolactone, γ-butyrolactone (GBL), N-methyl-2-pyrrolidone (NMP), and mixtures thereof. In the present invention, the preferred anode is lithium metal and the preferred electrolyte is 0.8M to 1.5M LiAsF₆ or LiPF₆ dissolved in a 50:50 mixture, by volume, of propylene carbonate and 1,2-dimethoxyethane.

The corrosion resistant glass used in the glass-to-metal seals has up to about 50% by weight silicon such as CABAL 12, TA 23, FUSITE 425 or FUSITE 435. The positive terminal leads preferably comprise molybdenum, although titanium, aluminum, nickel alloy, or stainless steel can also be used. The cell casing is an open container of a conductive material selected from nickel, aluminum, stainless steel, mild steel, tantalum and titanium. The casing is hermetically sealed with a lid, typically of a material similar to that of the casing.

The coated-SVO and CSVO particles are particularly useful in electrochemical cells containing lithium anodes and non-aqueous electrolytes. In a typical cell, the cathode consists of a mixture of, by weight, about 94% coated-SVO along with 3% PTFE, 2% graphite and 1% carbon black. The cathode is separated from the lithium anode by a layer of polypropylene separator. The cell is activated with 1 M LiAsF₆ in PC/DME (1:1) electrolyte. Pulse testing of the cell is accomplished by subjected it to high current pulses (-23 mA/cm²) for 10 seconds in duration. The current pulses are applied in groups of four, with 15 seconds of rest between pulses. Time between application of the pulse groups ranges from several weeks to six months. Total discharge time for the cell is up to ten years. This makes the cell particularly well suited for powering an implantable medical device, such as a cardiac pacemaker, cardiac defibrillator, drug pump, neurostimulator, self-contained artificial heart, and the like.

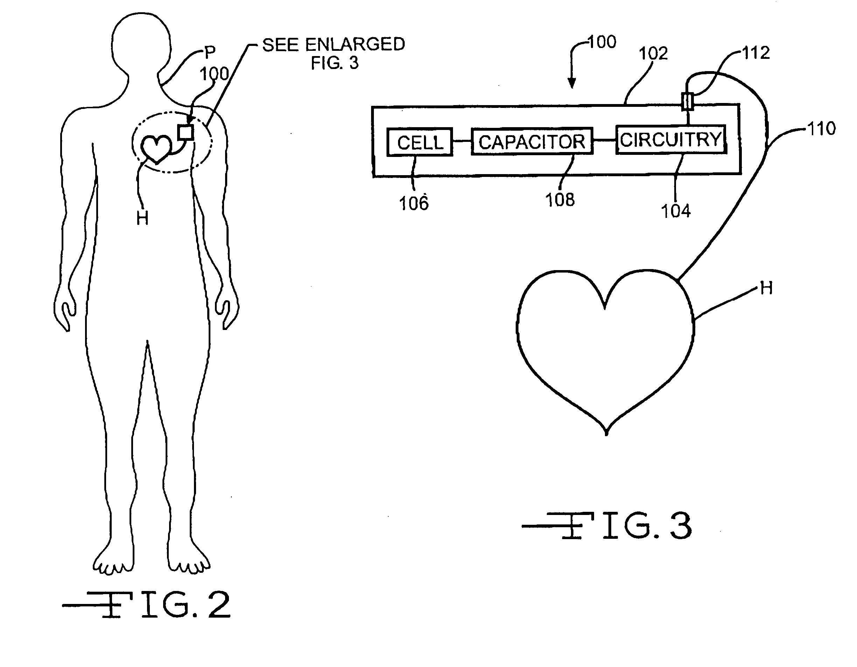

Figs. 2 and 3 show a patient P having a medical device 100, such as an implantable cardiac defibrillator, implanted inside the body. The enlarged schematic shows the medical device 100 comprising a housing 102 containing control circuitry 104 powered by an electrochemical cell 106 according to the present invention. The cell 106 is also connected to a capacitor 108. The control circuitry 104 is connected to at least one conductor 110 by a hermetic feedthrough 112, as is well known by those skilled in the art. The distal end of the conductor connects to the heart H for delivering a therapy thereto from the capacitor 108 charged by the cell 106.

Periodically, the patient will go to a medical facility, and the like, where the deliverable capacity determined by the control circuitry 104 is read to determine if the cell 106 has discharged to the point that it is approaching its end-of-life, typically at an open circuit voltage of about 2.0 volts. If so, this indicates that it is time for the physician to schedule the patient for surgery to replace the medical device with a new one.

It is appreciated that various modifications to the inventive concepts described herein may be apparent to those of ordinary skill in the art without departing from the spirit and scope of the present invention as defined by the appended claims.

## Claims

1. An electrochemical cell, which comprises:
a) an anode of an alkali metal;
b) a cathode of a cathode active material provided with a coating having the formula MₓO_{y}, wherein x = 1 or 2 and y = 1 to 3, or the formula LiₓM_{y}O_{z} wherein x = 1, y = 1 or 2 and z = 1 to 4, and mixtures thereof; and
c) a nonaqueous electrolyte activating the anode and the cathode.

2. The electrochemical cell of claim 1, wherein M in the coating formulas of MₓO_{y} and LiₓM_{y}O_{z}, is selected from the group consisting of Al, B, Mg, Mn, Si, Sn, Zr, and mixtures thereof.

3. The electrochemical cell of claim 1 or claim 2, wherein the coating is selected from the group consisting of SnO₂, SiO₂, Al₂O₃, ZrO₂, B₂O₃, MgO, MnO₂, LiCoO₂, LiMnₓO_{y}, and mixtures thereof .

4. The electrochemical cell of any of claims 1 to 3, wherein the cathode active material is selected from the group consisting of SVO, CSVO, V₂O₅, MnO₂, LiCoO₂, LiNiO₂, LiMnO₂, LiMn₂O₄, CuO₂, TiS₂, Cu₂S, FeS, FeS₂, Ag₂O, Ag₂O₂, CuF, Ag₂CrO₄, copper vanadium oxide, and mixtures thereof.

5. The electrochemical cell of any of claims 1 to 4, wherein the cathode active material is contacted to a cathode current collector selected from the group consisting of stainless steel, titanium, tantalum, platinum, aluminum, gold, nickel, and alloys thereof, preferably wherein the cathode active material is contacted to a titanium cathode current collector having a graphite/carbon material coated thereon.

6. The electrochemical cell of any of claims 1 to 5, wherein the anode is lithium and the cathode active material is SVO having its individual particles provided with a coating selected from the group consisting of SnO₂, SiO₂, Al₂O₃, ZrO₂, B₂O₃, MgO, MnO₂, LiCoO₂, LiMnOₓ, and mixtures thereof.

7. The electrochemical cell of any of claims 1 to 6, built in one of a case-negative design, a case-positive design and a case-neutral design.

8. The electrochemical cell of claim 1 wherein the electrolyte has a first solvent selected from an ester, a linear ether, a cyclic ether, a dialkyl carbonate, and mixtures thereof, and a second solvent selected from a cyclic carbonate, a cyclic ester, a cyclic amide, and mixtures thereof, preferably wherein the first solvent is selected from the group consisting of tetrahydrofuran, methyl acetate, diglyme, triglyme, tetraglyme, dimethyl carbonate, 1,2-dimethoxyethane, 1,2-diethoxyethane, 1-ethoxy,2-methoxyethane, ethyl methyl carbonate, methyl propyl carbonate, ethyl propyl carbonate, diethyl carbonate, dipropyl carbonate, and mixtures thereof, and the second solvent is selected from the group consisting of propylene carbonate, ethylene carbonate, butylene carbonate, acetonitrile, dimethyl sulfoxide, dimethyl formamide, dimethyl acetamide, Y-valerolactone, Y-butyrolactone, N-methyl-2-pyrrolidone, and mixtures thereof.

9. The electrochemical cell of any of claims 1 to 8, wherein the electrolyte includes a lithium salt selected from the group consisting of LiPF₆, LiBF₄, LiAsF₆, LiSbF₆, LiClO₄, LiO₂, LiAlCl₄, LiGaCl₄, LiC(SO₂CF₃)₃, LiN(SO₂CF₃)₂, LiSCN, LiO₃SCF₃, LiC₆F₅SO₃, LiO₂CCF₃, LiSO₆F, LiB (C₆H₅)₄, LiCF₃SO₃, and mixtures thereof.

10. An implantable medical device (100), which comprises:
a) a device housing (102);
b) control circuitry (104) contained inside the device housing (102);
c) an electrochemical cell (106) housed inside the device housing (102) for powering the control circuitry (104), the cell (106) comprising:
i) an anode comprising lithium;
ii) a cathode of silver vanadium oxide provided with a coating having the formula MₓO_{y}, wherein x = 1 or 2 and y = 1 to 3 or the formula LiₓM_{y}O_{z}, wherein x = 1, y = 1 or and z = 1 to 4, and mixtures thereof; and
iii) a nonaqueous electrolyte activating the anode and the cathode; and
d) a lead connecting the device housing (102) to a body part intended to be assisted by the medical device (100), wherein the electrochemical cell (106) powers the control circuitry (104) both during a device monitoring mode to monitor the physiology of the body part and a device activation mode to provide the therapy to the body part.

11. The implantable medical device (100) of claim 10, wherein M in the coating formulas of MₓO_{y} and LiₓM_{y}O_{z} is selected from the group consisting of Al, B, Mg, Mn, Si, Sn, Zr, and mixtures thereof.

12. The implantable medical device (100) of claim 10 or claim 11, wherein the coating is selected from the group consisting of SnO₂, SiO₂, Al₂O₃, ZrO₂, B₂O₃, MgO, MnO₂, LiCoO₂, LiMnₓO_{y}, and mixtures thereof.

13. The implantable medical device (100) of any of claims 10 to 12, wherein the cathode active material is selected from the group consisting of SVO, CSVO, V₂O₅, MnO₂, LiCoO₂, LiNiO₂, LiMnO₂, LiMn₂O₄, CuO₂, TiS₂, Cu₂S, FeS, FeS₂, Ag₂O, Ag₂O₂, CuF, Ag₂CrO₄, copper vanadium oxide, and mixtures thereof.

14. The implantable medical device (100) of any of claims 10 to 13, wherein the cathode active material is contacted to a cathode current collector selected from the group consisting of stainless steel, titanium, tantalum, platinum, aluminum, gold, nickel, and alloys thereof, preferably wherein the cathode active material is contacted to a titanium cathode current collector having a graphite/carbon material coated thereon.

15. The implantable medical device (100) of any of claims 10 to 14, wherein the anode is lithium and the cathode active material is SVO having its individual particles provided with a coating selected from the group consisting of SnO₂, SiO₂, Al₂O₃, ZrO₂, B₂O₃, MgO, MnO₂, LiCoO₂, LiMnₓO_{y}, and mixtures thereof.

16. A method for providing a cathode active material, comprising the steps of:
a) providing the cathode active material is granular form;
b) providing a sol-gel solution of an organic solvent having a coating metal selected from Al, B, Mg, Mn, Si, Sn, Zr, and mixtures thereof provided therein;
c) mixing the cathode active material into the sol-gel solution;
d) drying the resulting coated cathode active material to substantially remove the solvent material;
e) heating the dried coated active material to convert the coating metal to a coating having the formula MₓO_{y}, wherein x = 1 or 2 and y = 1 to 3, or the formula LiₓM_{y}O_{z} wherein x = 1, y = 1 or and 2 = 1 to 4, and mixtures thereof.

17. The method of claim 16, wherein the coating is selected from the group consisting of SnO₂, SiO₂, Al₂O₃, ZrO₂, B₂O₃, MgO, MnO₂, LiCoO₂, LiMnₓO_{y}, and mixtures thereof.

18. The method of claim 16 or claim 17, including selecting the cathode active material from the group consisting of SVO, CSVO, V₂O₅, MnO₂, LiCoO₂, LiNiO₂, LiMnO₂, LiMn₂O₄, CuO₂, TiS₂, Cu₂S, FeS, FeS₂, Ag₂O, Ag₂O₂, CuF, Ag₂CrO₄, copper vanadium oxide, and mixtures thereof.

19. The method of any of claims 16 to 18, including providing the sol-gel solution as either an aqueous or a nonaqueous solution.

20. The method of any of claims 16 to 19, including mixing the coating metal with the active material in a range, by weight, of 1 : 3 to 1 : 20.

21. The method of any of claims 16 to 20, including drying the coated cathode active material at a reduced pressure in a range of 675.6 mbar (20 inches of Hg) to 1689.1 mbar (50 inches of Hg).

22. The method of any of claims 16 to 21, including drying the coated cathode active material at a temperature in a range of 200 °C to 500 °C and/or including drying the coated cathode active material for a time of 10 minutes to 6 hours.
